Europäisches Patentamt

European Patent Office

Office européen des brevets

(11) Publication number: **0 331 489 B1**

# EUROPEAN PATENT SPECIFICATION

(45) Date of publication of patent specification: **07.07.93** (51) Int. Cl.⁵: **A61K 7/48**, A61K 47/00

(21) Application number: **89302081.8**

(22) Date of filing: **02.03.89**

The file contains technical information submitted after the application was filed and not included in this specification

(54) **Acne treatment.**

(30) Priority: **03.03.88 AU 511/88**
     **20.12.88 AU 2046/88**

(43) Date of publication of application:
     **06.09.89 Bulletin 89/36**

(45) Publication of the grant of the patent:
     **07.07.93 Bulletin 93/27**

(84) Designated Contracting States:
     **AT BE CH DE ES FR GB GR IT LI LU NL SE**

(56) References cited:
     **AU-A- 463 216**
     **GB-A- 2 018 589**
     **GB-A- 2 181 349**
     **US-A- 3 535 422**

(73) Proprietor: **PARKE DAVIS PTY. LTD.**
     **32 Cawarra Road**
     **Caringbah New South Wales, 2229(AU)**

     Proprietor: **SOLTEC RESEARCH PTY. LTD.**
     **8 Macro Court**
     **Rowville Victoria 3178(AU)**

(72) Inventor: **Tomlinson, Roderick Peter**
     **32 Highwood Drive**
     **Glen Waverley Victoria, 3150(AU)**

(74) Representative: **Jones, Michael Raymond et al**
     **HASELTINE LAKE & CO. Hazlitt House 28**
     **Southampton Buildings Chancery Lane**
     **London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

**EP 0 331 489 B1**

**Description**

The present invention resides in a new bacteriacidal composition which is particularly applicable for use in treatment of acne.

Acne is caused by bacterial infection.

In the past, treatment of acne has normally comprised the external application of solutions, lotions or creams containing benzoyl peroxide or such phenolics as resorcinol. Although such treatment may be effective in some instances, the high proportion of instances where external treatment has been largely ineffective has led to the development of internal treatments, administration of which is liable to cause serious side-effects and is therefore highly undesirable except in very extreme cases.

Consequently, it has long been recognised that development of new methods of treating acne is highly desirable.

US-A-3535422 is concerned with providing storage stable compositions for the treatment of acne which contain benzoyl peroxide. This document describes compositions containing benzoyl peroxide, water and an organic emollient which may be a saturated aliphatic alcohol, a glycol, a saturated fatty acid, a lower alkyl or glyceryl ester of a fatty acid or a mixture thereof. The compositions are dermally applied in the form of creams or ointments.

It has been found that one of the main reasons for the ineffectiveness of the known solutions, lotions and creams is that the active constituents do not reach the appropriate dermal site where treatment is required. Solutions tend to evaporate before penetrating the epidermis, and lotions and creams are generally too viscous to allow penetration of the epidermis.

The present invention ameliorates at least some of the disadvantages of the prior art.

According to the present invention, there is provided a bactericidal composition for use in the external treatment of acne, said composition comprising:

(a) a biocide effective in killing acne bacteria;

(b) a fatty acid;

(c) a quick breaking foaming agent comprising an aliphatic alcohol, water, a fatty alcohol and a surface active agent; and

(d) an aerosol propellant.

Such compositions are of low viscosity and are therefore capable of rapid penetration of the epidermis. Preferred biocides are chosen from the group consisting of parachlorometraxylenol (PCMX), benzoyl peroxide, triclosan, sulphur and salicylic acid or mixtures thereof. The preferred fatty acids in the composition of the present invention are linoleic and/or linolenic acid although mixtures with other fatty acids of this type are also suitable. Linoleic and/or linolenic acid is preferably present in an amount of 0.5 - 5%w/w, more preferably of 0.82 - 1.25%w/w composition.

The structure of the composition is improved relative to conventional compositions as it is capable of forming a quick breaking form, thus reducing the amount of evaporation on application but at the same time allowing rapid penetration of the epidermis. Quick breaking foams minimise early evaporation as they are applied to the skin surface as a thick foam which rapidly disintegrates when spread.

One preferred composition which forms a quick breaking foam comprises biocide, linoleic and/or linolenic acid, the quick breaking foam agent and an aerosol propellant. Preferably a corrosion inhibitor is also included if the composition is to be stored in metal containers. However, this is not necessary if the container is of non-metal such as glass or plastic. The biocide is preferably in the composition in an amount between 0.05 and 1% w/w composition. Most preferably the amount is approximately 0.2%w/w.

Quick breaking foaming agents for producing quick breaking foams are well known. A general discussion may be found in Australian Patent AU-A-463216. The quick breaking foaming agent in the composition of the present invention comprises an aliphatic alcohol, water, a fatty alcohol and a surface active agent. Preferably, the aliphatic alcohol is aqueous ethanol.

The quick break foaming agent comprises:

(a) An aliphatic alcohol preferably in amounts from 40-90%w/w composition, more preferably 55-70%w/w and most preferably 60%w/w;

(b) Water preferably in amounts from 10-40%w/w;

(c) A fatty alcohol, preferably in amounts from 0.5-10%w/w; and

(d) A surface active agent preferably an ethoxylated sorbitan ester (as emulsifier) typically in amounts from 0.1-15% w/w.

Aerosol propellants for use in the compositions of the present invention should be carefully selected for their compatability with the entire composition. The most preferred propellants may be selected from the group comprising propane, butane, dichloro difluoro methane, dichloro tetra fluoro ethane, and octafluoro

2

cyclo butane. The propellant is preferably present in amounts from 3 to 30% w/w, more preferably from 5-15%w/w and most preferably 8 to 10% w/w.

Preferred corrosion inhibitors for use in the present invention include organic acid salts such as sorbic acid, benzoic acid, sodium benzoate and potassium sorbate. The preferred amount of corrosion inhibitor is 0.1 to 15%w/w, more preferably 0.1 to 3%w/w.

The invention will now be more fully described by way of the following examples.

Example 1.

The following formulation is a general formulation which may be used as a basis for more specific formulations thus enabling variation as required for particular circumstances.

|  | %w/w |
|---|---|
| PROPELLANT e.g. propane, butane, dichloro difluoro methane, dichloro tetra fluoro ethane, octafluoro cyclo butane and mixtures thereof | 3-30 |
| BIOCIDE e.g. parachlorometaxylenol, Benzoyl Peroxide, Triclosan, Sulphur, Salicylic Acid and mixtures thereof | 0.05-10 |
| FATTY ACID e.g. linoleic and/or linolenic acid | 0.5-5 |
| FATTY ALCOHOL e.g. cetyl, stearyl, lauryl, myristyl, palmityl, and mixtures thereof | 0.5-10 |
| ALIPHATIC ALCOHOL e.g. methyl, ethyl isopropyl, butyl and mixtures thereof | 40-90 |
| WATER | 10-40 |
| POLYOL e.g. glycerol, propylene glycol, sorbitol and low molecular weight polymers thereof | 1-10 |
| ORGANIC ACID SALT e.g. sorbic acid, benzoic acid | 0.1-15 |
| SURFACE ACTIVE AGENT e.g. ethoxylated sorbitan stearate, palmitate, oleate, nonyl phenol ethoxylates, fatty alcohol ethoxylates. | 0.1-15 |

The following examples are particularly preferred embodiments of the present invention.

Example 2.

|  | %w/w |
|---|---|
| cetostearyl alcohol B.P. | 1.60 |
| polyoxyethylene sorbitan monostearate N.F. | 0.40 |
| ethanol 95% U.S.P. | 52.50 |
| polyunsaturated fatty acid | 1.85 |
| purified water B.P. | 30.90 |
| sodium hydroxide B.P | 0.25 |
| parachlorometaxylenol | 0.20 |
| sodium benzoate B.P. | 1.20 |
| propylene glycol B.P. | 2.00 |
| fragrance | 0.10 |
| F12/114 (40:60) B.P. | 9.00 |
|  | 100.00 |

3

Example 3.

| | %w/w |
|---|---|
| Parachlorometaxylenol | 0.20 |
| linoleic acid | 1.25 |
| cetyl stearyl alcohol | 2.50 |
| ethoxylated sorbitan monostearate | 0.50 |
| propylene glycol | 3.00 |
| ethyl alcohol (95%) | 57.00 |
| sodium benzoate | 0.20 |
| purified water | 26.35 |
| dichloro difluoro methane )<br>dichloro tetrafluoro ethane) blend | 9.00 |
| | 100.00 |

Example 4.

| | %w/w |
|---|---|
| Parachlorometaxylenol | 0.18 |
| linolenic acid | 0.82 |
| myristyl alcohol | 3.00 |
| ethoxylated octylalcohol | 0.80 |
| glycerol | 2.5 |
| isopropyl alcohol | 60.0 |
| potassium sorbate | 0.3 |
| purified water | 25.4 |
| butane propane | 7.0 |
| | 100.00 |

4

Example 5.

|  | %w/w |
|---|---|
| Sulphur | 0.5 |
| linoleic acid | 1.5 |
| myristyl alcohol | 3.0 |
| glycerol | 2.5 |
| ethoxylated myristyl alcohol | 0.5 |
| ethyl alcohol 95% | 58.0 |
| potassium sorbate | 1.0 |
| purified water | 23.0 |
| dichloro difluoro methane ) | |
| dichloro tetrafluoro ethane) | 10.00 |
| | 100.00 |

Example 6.

|  | %w/w |
|---|---|
| cetostearyl alcohol B.P. | 1.60 |
| polyoxyethylene sorbitan monostearate N.F. | 0.40 |
| ethanol 95% U.S.P. | 52.50 |
| polyunsaturated fatty acid | 1.85 |
| purified water B.P. | 30.90 |
| sodium hydroxide B.P | 0.25 |
| salicylic acid | 0.20 |
| sodium benzoate B.P. | 1.20 |
| propylene glycol B.P. | 2.00 |
| fragrance | 0.10 |
| F12/114 (40:60) B.P. | 9.00 |
| | 100.00 |

Example 7.

| | %w/w |
|---|---|
| Salicylic acid | 0.20 |
| linoleic acid | 1.25 |
| cetyl stearyl alcohol | 2.50 |
| ethoxylated sorbitan monostearate | 0.50 |
| propylene glycol | 3.00 |
| ethyl alcohol (95%) | 57.00 |
| sodium benzoate | 0.20 |
| purified water | 26.35 |
| dichloro difluoro methane ) | |
| dichloro tetrafluoro ethane) blend | 9.00 |
| | 100.00 |

Example 8.

| | %w/w |
|---|---|
| Triclosan | 0.18 |
| linolenic acid | 0.82 |
| myristyl alcohol | 3.00 |
| ethoxylated octylalcohol | 0.80 |
| glycerol | 2.50 |
| isopropyl alcohol | 60.00 |
| potassium sorbate | 0.30 |
| purified water | 25.40 |
| butane propane | 7.00 |
| | 100.00 |

Example 9.

| | %w/w |
|---|---|
| Sulphur | 0.50 |
| linoleic acid | 1.50 |
| myristyl alcohol | 3.00 |
| glycerol | 2.50 |
| ethoxylated myristyl alcohol | 0.50 |
| ethyl alcohol 95% | 58.00 |
| potassium sorbate | 1.00 |
| purified water | 23.00 |
| dichloro difluoro methane ) | |
| dichloro tetrafluoro ethane) | 10.00 |
| | 100.00 |

Example 10.

| | %w/w |
|---|---|
| Benzoyl Peroxide | 0.50 |
| linoleic acid | 1.50 |
| myristyl alcohol | 3.00 |
| glycerol | 2.50 |
| ethoxylated myristyl alcohol | 0.50 |
| ethyl alcohol 95% | 58.00 |
| potassium sorbate | 1.00 |
| purified water | 23.00 |
| dichloro difluoro methane ) | |
| dichloro tetrafluoro ethane) | 10.00 |
| | 100.00 |

Example 11.

EP 0 331 489 B1

| | %w/w |
|---|---|
| cetostearyl alcohol B.P. | 1.60 |
| polyoxyethylene sorbitan monostearate N.F. | 0.40 |
| ethanol 95% U.S.P. | 53.75 |
| polyunsaturated fatty acid | 1.85 |
| purified water B.P. | 34.65 |
| sodium hydroxide B.P. | 0.25 |
| parachlorometaxylenol | 0.20 |
| sodium benzoate B.P. | 1.20 |
| propylene glycol B.P. | 2.00 |
| fragrance | 0.10 |
| propane/butane | 4.00 |
| | 100.00 |

Example 12.

| | %w/w |
|---|---|
| cetostearyl alcohol B.P. | 2.10 |
| polyoxyethylene sorbitan monostearate N.F. | 0.40 |
| ethanol 95% U.S.P. | 53.40 |
| polyunsaturated fatty acid | 1.85 |
| purified water B.P. | 34.50 |
| sodium hydroxide B.P. | 0.25 |
| parachlorometaxylenol | 0.20 |
| sodium benzoate B.P. | 1.20 |
| propylene glycol B.P. | 2.00 |
| fragance | 0.10 |
| propane/butane | 4.00 |
| | 100.00 |

**Claims**
**Claims for the following Contracting States : AT, BE, CH, DE, FR, GB, IT, LI, LU, NL, SE**

1. A bactericidal composition for use in the external treatment of acne, said composition comprising:
   (a) a biocide effective in killing acne bacteria;
   (b) a fatty acid;
   (c) a quick breaking foaming agent comprising an aliphatic alcohol, water, a fatty alcohol and a surface active agent; and
   (d) an aerosol propellant.

2. A composition according to claim 1, wherein the agent comprises from 40 to 90%w/w of the aliphatic alcohol, from 10% to 40%w/w of water, from 0.5 to 10%w/w of the fatty alcohol and from 0.1 to 15%w/w of the surface active agent.

3. A composition according to either of the preceding claims, wherein the biocide is selected from parachlorometaxylenol, benzoyl peroxide, triclosan, sulphur, salicylic acid, or mixtures thereof.

4. A composition according to any one of the present claims, wherein the fatty acid is selected from linoleic acid, linolenic acid, or a mixture thereof.

5. A composition according to any one of the present claims, wherein the aliphatic alcohol is aqueous ethanol.

8

6. A composition according to any one of the preceding claims, wherein the composition is in the form of a quick breaking foam.

7. A composition according to any one of the preceding claims, comprising from 0.05 to 10%w/w of a biocide selected from parachlorometaxylenol, benzoyl peroxide, triclosan, sulphur, salicylic acid or mixtures thereof, from 0.5 to 5.0%w/w of linoleic and/or linolenic acid, from 24.0 to 95.25%w/w of said quick breaking foaming agent, and from 3 to 30%w/w of an aerosol propellant.

8. A composition according to any one of the preceding claims, which further comprises a corrosion inhibitor.

9. A composition according to any one of the preceding claims, wherein the biocide is present in an amount of approximately 0.2%w/w.

10. A composition according to any one of claims 4 to 10, wherein the linoleic and/or linolenic acid is present in an amount between 0.8 and 1.25%w/w.

11. A composition according to any one of the preceding claims for use in the manufacture of a medicament for the external treatment of acne.

**Claims for the following Contracting States : ES, GR**

1. A process for the preparation of a bactericidal composition for use in the external treatment of acne, which process comprises admixing:
   (a) a biocide effective in killing acne bacteria;
   (b) a fatty acid;
   (c) a quick breaking foaming agent comprising an aliphatic alcohol, water, a fatty alcohol and a surface active agent; and
   (d) an aerosol propellant.

2. A process according to claim 1, wherein the quick breaking foaming agent comprises from 40 to 90%w/w of the aliphatic alcohol, from 10 to 40%w/w of water, from 0.5 to 10%w/w of the fatty alcohol and from 0.1 to 15%w/w of the surface active agent.

3. A process according to either of the preceding claims, wherein the biocide is selected from parachlorometaxylenol, benzoyl peroxide, triclosan, sulphur, salicylic acid, or mixtures thereof.

4. A process according to any one of the preceding claims, wherein the fatty acid is selected from linoleic acid, linolenic acid, or a mixture thereof.

5. A process according to any one of the preceding claims, wherein the aliphatic alcohol is aqueous ethanol.

6. A process according to any one of the preceding claims, wherein the composition is formed into a quick breaking foam.

7. A process according to any one of the preceding claims, in which the composition comprises from 0.05 to 10%w/w of a biocide selected from parachlorometaxylenol, benzoyl peroxide, triclosan, sulphur, salicylic acid or mixtures thereof, from 0.5 to 5.0%w/w of linoleic and/or linolenic acid, from 24.0 to 95.25%w/w of said quick breaking foaming agent, and from 3 to 30%w/w of an aerosol propellant.

8. A process according to any one of the preceding claims, which further comprises admixing a corrosion inhibitor.

9. A process according to any one of the preceding claims, wherein the biocide is present in an amount of approximately 0.2%w/w.

9

**10.** A process according to any one of claims 4 to 9, wherein the linoleic and/or linolenic acid is present in an amount between 0.8 and 1.25%w/w.

**Patentansprüche**

**Patentansprüche für folgende Vertragsstaaten : AT, BE, CH, DE, FR, GB, IT, LI, LU, NL, SE**

**1.** Bakterizide Zusammensetzung zur Verwendung bei der äußerlichen Behandlung von Akne, wobei diese Zusammensetzung enthält: (a) ein zum Abtöten von Aknebakterien wirksames Biozid; (b) eine Fettsäure; (c) ein schnellbrechendes Schaummittel, umfassend einen aliphatischen Alkohol, Wasser, einen Fettalkohol und ein oberflächenaktives Mittel; und (d) ein Aerosoltreibmittel.

**2.** Zusammensetzung nach Anspruch 1, worin das Mittel 40 bis 90 Gew.-% des aliphatischen Alkohols, 10 bis 40 Gew.-% Wasser, 0,5 bis 10 Gew.-% des Fettalkohols und 0,1 bis 15 Gew.-% des oberflächenaktiven Mittels umfaßt.

**3.** Zusammensetzung nach einem der vorhergehenden Ansprüche, worin das Biozid aus Parachlormetaxylenol, Benzoylperoxid, Triclosan, Schwefel, Salicylsäure oder deren Gemischen ausgewählt wird.

**4.** Zusammensetzung nach irgendeinem der vorhergehenden Ansprüche, worin die Fettsäure aus Linolsäure, Linolensäure oder einem Gemisch davon ausgewählt wird.

**5.** Zusammensetzung nach irgendeinem der vorhergehenden Ansprüche, worin der aliphatische Alkohol wäßriges Ethanol ist.

**6.** Zusammensetzung nach irgendeinem der vorhergehenden Ansprüche, worin die Zusammensetzung in Form eines schnellbrechenden Schaums vorliegt.

**7.** Zusammensetzung nach irgendeinem der vorhergehenden Ansprüche, umfassend 0,05 bis 10 Gew.-% eines aus Parachlormetaxylenol, Benzoylperoxid, Triclosan, Schwefel, Salicylsäure oder deren Gemischen ausgewählten Biozids, 0,5 bis 5,0 Gew.-% Linolsäure und/oder Linolensäure, 24,0 bis 95,25 Gew.-% des schnellbrechenden Schaummittels und 3 bis 30 Gew.-% eines Aerosoltreibmittels.

**8.** Zusammensetzung nach irgendeinem der vorhergehenden Ansprüche, welche weiters einen Korrosionsinhibitor enthält.

**9.** Zusammensetzung nach irgendeinem der vorhergehenden Ansprüche, worin das Biozid in einer Menge von ungefähr 0,2 Gew.-% vorliegt.

**10.** Zusammensetzung nach irgendeinem der Ansprüche 4 bis 9, worin die Linolsäure und/oder Linolensäure in einer Menge zwischen 0,8 und 1,25 Gew.-% vorliegt.

**11.** Zusammensetzung nach irgendeinem der vorhergehenden Ansprüche zur Verwendung bei der Herstellung eines Medikamentes zur äußerlichen Behandlung von Akne.

**Patentansprüche für folgende Vertragsstaaten : ES, GR**

**1.** Verfahren zur Herstellung einer bakteriziden Zusammensetzung zur Verwendung bei der äußerlichen Behandlung von Akne, wobei dieses Verfahren das Vermischen von (a) einem zum Abtöten von Aknebakterien wirksamen Biozid; (b) einer Fettsäure; (c) einem schnellbrechenden Schaummittel, umfassend einen aliphatischen Alkohol, Wasser, einen Fettalkohol und ein oberflächenaktives Mittel; und (d) einem Aerosoltreibmittel umfaßt.

**2.** Verfahren nach Anspruch 1, worin das schnellbrechende Schaummittel 40 bis 90 Gew.-% des aliphatischen Alkohols, 10 bis 40 Gew.-% Wasser, 0,5 bis 10 Gew.-% des Fettalkohols und 0,1 bis 15 Gew.-% des oberflächenaktiven Mittels umfaßt.

**3.** Verfahren nach einem der vorhergehenden Ansprüche, worin das Biozid aus Parachlormetaxylenol, Benzoylperoxid, Triclosan, Schwefel, Salicylsäure oder deren Gemischen ausgewählt wird.

EP 0 331 489 B1

**4.** Verfahren nach irgendeinem der vorhergehenden Ansprüche, worin die Fettsäure aus Linolsäure, Linolensäure oder einem Gemisch davon ausgewählt wird.

**5.** Verfahren nach irgendeinem der vorhergehenden Ansprüche, worin der aliphatische Alkohol wäßriges Ethanol ist.

**6.** Verfahren nach irgendeinem der vorhergehenden Ansprüche, worin die Zusammensetzung zu einem schnellbrechenden Schaum gebildet wird.

**7.** Verfahren nach irgendeinem der vorhergehenden Ansprüche, worin die Zusammensetzung 0,05 bis 10 Gew.-% eines aus Parachlormetaxylenol, Benzoylperoxid, Triclosan, Schwefel, Salicylsäure oder deren Gemischen ausgewählten Biozids, 0,5 bis 5,0 Gew.-% Linolsäure und/oder Linolensäure, 24,0 bis 95,25 Gew.-% des schnellbrechenden Schaummittels und 3 bis 30 Gew.-% eines Aerosoltreibmittels enthält.

**8.** Verfahren nach irgendeinem der vorhergehenden Ansprüche, welches weiters das Zumischen eines Korrosionsinhibitors umfaßt.

**9.** Verfahren nach irgendeinem der vorhergehenden Ansprüche, worin das Biozid in einer Menge von ungefähr 0,2 Gew.-% vorliegt.

**10.** Verfahren nach irgendeinem der Ansprüche 4 bis 9, worin die Linolsäure und/oder Linolensäure in einer Menge zwischen 0,8 und 1,25 Gew.-% vorliegt.

**Revendications**

**Revendications pour les Etats contractants suivants : AT, BE, CH, DE, FR, GB, IT, LI, LU, NL, SE**

**1.** Composition bactéricide à utiliser dans le traitement externe de l'acné, ladite composition comprenant :
(a) un biocide efficace pour détruire les bactéries de l'acné ;
(b) un acide gras ;
(c) un agent moussant à résolution rapide comprenant un alcool aliphatique, de l'eau, un alcool gras et un agent tensioactif ; et
(d) un pulseur d'aérosol.

**2.** Composition selon la revendication 1, dans laquelle l'agent comprend de 40 à 90% p/p d'alcool aliphatique, de 10% à 40% p/p d'eau, de 0,5 à 10% p/p d'alcool gras et de 0,1 à 15% p/p d'agent tensioactif.

**3.** Composition selon l'une quelconque des revendications précédentes, dans laquelle le biocide est sélectionné parmi le parachlorométhaxylénol, le peroxyde de benzoyle, le triclosan, le soufre, l'acide salicylique ou leurs mélanges.

**4.** Composition selon l'une quelconque des revendications précédentes, dans laquelle l'acide gras est sélectionné parmi l'acide linoléique, l'acide linolénique ou leur mélange.

**5.** Composition selon l'une quelconque des revendications précédentes, dans laquelle l'alcool aliphatique est de l'éthanol aqueux.

**6.** Composition selon l'une quelconque des revendications précédentes, dans laquelle la composition se présente sous la forme d'une mousse à résolution rapide.

**7.** Composition selon l'une quelconque des revendications précédentes, comprenant de 0,05 à 10% p/p d'un biocide sélectionné parmi le parachlorométaxylénol, le peroxyde de benzoyle, le triclosan, le soufre, l'acide salicylique ou leurs mélanges, de 0,5 à 5,0% p/p d'acide linoléique et/ou d'acide linolénique, de 24,0 à 95,25% p/p dudit agent moussant à résolution rapide et de 3 à 30% p/p d'un pulseur d'aérosol.

**8.** Composition selon l'une quelconque des revendications précédentes, qui comprend en outre un inhibiteur de corrosion.

11

**9.** Composition selon l'une quelconque des revendications précédentes, dans laquelle le biocide est présent en une quantité approximative de 0,2% p/p.

**10.** Composition selon l'une quelconque des revendications 4 à 10, dans laquelle l'acide linoléique et/ou linolénique est présent en une quantité comprise entre 0,8 et 1,25% p/p.

**11.** Composition selon l'une quelconque des revendications précédentes, à utiliser dans la préparation d'un médicament destiné au traitement externe de l'acné.

**Revendications pour les Etats contractants suivants : ES, GR**

**1.** Procédé de préparation d'une composition bactéricide à utiliser dans le traitement externe de l'acné, lequel procédé comprend le mélange de :
   (a) un biocide efficace pour détruire les bactéries de l'acné ;
   (b) un acide gras ;
   (c) un agent moussant à résolution rapide comprenant un alcool aliphatique, de l'eau, un alcool gras et un agent tensioactif ; et
   (d) un pulseur d'aérosol.

**2.** Procédé selon la revendication 1, dans lequel l'agent moussant à résolution rapide comprend de 40 à 90% p/p d'alcool aliphatique, de 10 à 40% p/p d'eau, de 0,5 à 10% p/p d'alcool gras et de 0,1 à 15% p/p d'agent tensioactif.

**3.** Procédé selon l'une quelconque des revendications précédentes, dans lequel le biocide est sélectionné parmi le parachlorométaxylénol, le peroxyde de benzoyle, le triclosan, le soufre, l'acide salicylique ou leurs mélanges.

**4.** Procédé selon l'une quelconque des revendications précédentes, dans lequel l'acide gras est sélectionné parmi l'acide linoléique, l'acide linolénique ou leur mélange.

**5.** Procédé selon l'une quelconque des revendications précédentes, dans lequel l'alcool aliphatique est de l'éthanol aqueux.

**6.** Procédé selon l'une quelconque des revendications précédentes, dans lequel la composition est formée en une mousse à résolution rapide.

**7.** Procédé selon l'une quelconque des revendications précédentes dans lequel la composition comprend de 0,05 à 10% p/p d'un biocide sélectionné parmi le parachlorométaxylénol, le peroxyde de benzoyle, le triclosan, le soufre, l'acide salicylique ou leurs mélanges, de 0,5 à 5,0% p/p d'acide linoléique et/ou linolénique, de 24,0 à 95,25% p/p dudit agent moussant à résolution rapide et de 3 à 30% p/p d'un pulseur d'aérosol.

**8.** Procédé selon l'une quelconque des revendications précédentes, lequel comprend en outre l'addition d'un inhibiteur de corrosion.

**9.** Procédé selon l'une quelconque des revendications précédentes, dans lequel le biocide est présent en une quantité approximative de 0,2% p/p.

**10.** Procédé selon l'une quelconque des revendications 4 à 9, dans lequel l'acide linoléique et/ou linolénique est présent en une quantité comprise entre 0,8 et 1,25% p/p.